# DEMANDE DE BREVET EUROPEEN

(11) **EP 1 195 144 A1**
(43) Date de publication de la demande: **10.04.2002**
(21) Numéro de dépôt: 01402560.5
(22) Date de dépôt: 04.10.2001
(51) Int. Cl.: A61B 17/86, A61B 17/88

(54) **Dispositif de vis à module d'insertion cassable pour des techniques d'ostéosynthèse**

(30) Priorité: 05.10.2000 FR 0012716
(71) Demandeur: Euro.Medic., 76120 Le Grand Quevilly (FR)
(72) Inventeur: Adrian, Denis, 76130 Mont Saint-Aignan (FR); Legay, Philippe, 76120 Le Grand Quevilly (FR)
(74) Mandataire: Thévenet, Jean-Bruno

(57) **Abrégé**

Le dispositif de vis à module d'insertion cassable (10) comprend une tige filetée implantable (4) raccordée par un étranglement (3) à une tige de préhension (1). La tige de préhension (1) comprend une portion (2) en forme de sifflet résistant à la torsion et raccordée à la tige filetée (4), dépourvue de tête de vis, au niveau de l'étranglement (3), de telle manière que la tige de préhension (1) puisse être cassée et séparée de la tige filetée (4) au niveau de l'étranglement (3) par simple flexion volontaire de la tige de préhension (1) par rapport à la tige filetée (4). Le dispositif est applicable aux techniques d'ostéosynthèse.

## Description

La présente invention a pour objet un dispositif de vis à module d'insertion cassable pour des techniques d'ostéosynthèse, comprenant une tige filetée implantable raccordée par un étranglement à une tige de préhension.

L'invention a également pour objet un outil d'extraction d'un dispositif de vis à module d'insertion cassable.

On connaît déjà, par exemple par les documents FR-A-2 625 430, FR-A-2 759 282 et FR-A-2 721 819, des dispositifs de vis ou de cheville auto-taraudeuse à embout de maintien sécable, pour la fixation d'une plaque d'ostéosynthèse ou la coaptation de deux fragments osseux.

De telles vis autocassables comprennent une tige filetée prolongée par une tête de vis qui est solidaire, par l'intermédiaire d'un étranglement de moindre résistance à un couple donné, d'une tige de préhension formant un embout d'adaptation dans le mandrin d'un outil de vissage.

Le principe de fonctionnement de telles vis autocassables repose sur la rupture en torsion (cisaillement), dès que la tête de la vis arrive en contrainte sur la corticale de l'os. Cet effet ne peut donc pas se produire lorsque l'on doit effectuer une exostosectomie ou lorsque l'os cortical est de mauvaise qualité.

Par ailleurs, dans les dispositifs de vis autocassables de l'art antérieur, la tête de vis a tendance à former une partie en relief par rapport à une plaque d'ostéosynthèse ou un fragment osseux. De plus, il est nécessaire de munir la tête de vis d'une ou plusieurs entailles ou empreintes périphériques pour permettre, soit de finir le vissage, soit de procéder à un dévissage pour retirer la vis après un certain temps d'utilisation. De telles empreintes sont la plupart du temps insuffisantes pour permettre une extraction aisée de la vis.

La présente invention vise à remédier aux inconvénients précités et notamment à faciliter la mise en place et l'extraction en toute sécurité d'une vis ou cheville à module d'insertion cassable dans le cadre de techniques d'ostéosynthèse, c'est-à-dire d'interventions chirurgicales permettant de réunir des fragments osseux d'une fracture par une pièce métallique afin d'assurer la consolidation par formation de cal.

Ces buts sont atteints grâce à un dispositif de vis à module d'insertion cassable pour des techniques d'ostéosynthèse, comprenant une tige filetée implantable raccordée par un étranglement à une tige de préhension, caractérisé en ce que la tige de préhension comprend une portion en forme de sifflet résistant à la torsion et raccordée, au niveau de l'étranglement, à la tige filetée dépourvue de tête de vis, de telle manière que la tige de préhension puisse être cassée et séparée de la tige filetée au niveau de l'étranglement par simple flexion volontaire de la tige de préhension par rapport à la tige filetée.

La portion en forme de sifflet est munie d'un biseau du côté raccordé à la tige filetée au niveau de l'étranglement.

Le sifflet peut présenter une forme symétrique ou dissymétrique par rapport à un plan axial de la vis, selon les applications envisagées.

Le sifflet peut présenter des parois inclinées d'un angle compris entre environ 15° et 30° par rapport à l'axe de la vis.

Dans le dispositif de vis selon l'invention, la rupture de la tige de préhension s'effectue dans tous les cas en flexion, et de façon volontaire, ce qui supprime tout risque de rupture inopinée ou d'absence de rupture susceptible d'entraîner la destruction de l'os proximal par perforation.

Selon un autre aspect de la présente invention, la tige filetée comprend au moins un filetage proximal croisé avec un premier filetage à droite, et un deuxième filetage à gauche formant contre-filet superposé au premier filetage au moins dans la partie voisine de l'étranglement, et un filetage distal au voisinage de la partie terminale pointue de la tige filetée.

Le filetage proximal croisé et le filetage distal sont différents.

Les filetages proximal et distal peuvent ainsi présenter des pas différents de manière à être compressifs.

Le filetage proximal croisé peut être de type à profil cortical tandis que le filetage distal est de type à profil spongieux.

Le deuxième filetage à gauche formant contre-filet peut s'étendre sur une partie seulement de la hauteur du premier filetage à droite, par exemple sensiblement selon la moitié de la hauteur du premier filetage à droite, ou selon une variante de réalisation, peut s'étendre sensiblement sur toute la hauteur du premier filetage à droite.

Selon une caractéristique particulière, la partie terminale pointue de la tige filetée comprend deux surfaces concaves délimitées par deux parois latérales définissant entre elles un angle de 90°.

La présence d'un filetage proximal croisé, droit et gauche, permet une bonne repousse osseuse sur le métal de la tige filetée et permet également d'extraire facilement la tige filetée implantée en vissant, en pas inverse, c'est à dire par dévissage sur le filetage à gauche, un guide de dévissage. Un tel guide de dévissage, dont la réalisation peut être très simple, est ainsi d'abord vissé sur le filetage à gauche de la vis (c'est à dire dans le sens de rotation d'un dévissage pour des filetages à droite). Arrivé en butée, le guide de dévissage entraîne la vis dans le même sens de rotation, c'est à dire en dévissage par rapport à l'os par action du filetage proximal à droite et du filetage distal à droite.

L'invention concerne également un outil d'extraction d'un dispositif de vis à module d'insertion cassable, caractérisé en ce qu'il comprend une tige d'extraction dont la portion distale constitue une partie tubulaire comprenant une partie lisse d'extrémité prolongée par un trou taraudé muni d'un filetage à gauche correspondant au deuxième filetage à gauche formant contre-filet de la vis à extraire, la partie tubulaire étant terminée par une extrémité dentelée et biseautée.

Le corps de la tige d'extraction peut présenter une section supérieure à la section de la partie tubulaire en étant relié à cette dernière par une partie tronconique.

L'outil d'extraction comprend à son extrémité opposée à la partie tubulaire, une partie d'emmanchement dans un mandrin d'entraînement en rotation.

D'une manière générale, une vis d'ostéosynthèse compressive selon l'invention est efficace même après exostosectomie et permet de choisir le moment précis de rupture de la tige d'introduction, tout en garantissant une extraction ultérieure aisée à l'aide d'un outil d'extraction de conception simple et efficace.

D'autres caractéristiques et avantages de l'invention ressortiront de la description suivante de modes particuliers de réalisation, donnés à titre d'exemples, en référence aux dessins annexés, sur lesquels :
- la figure 1 est une vue d'ensemble d'un dispositif de vis à module d'insertion cassable selon la présente invention,
- la figure 2 est une vue agrandie de la jonction entre la tige de préhension et la tige filetée du dispositif de vis de la figure 1,
- la figure 3 est une vue selon la flèche F de la figure 2, montrant la forme de sifflet de l'extrémité de la tige de préhension du dispositif de vis de la figure 1,
- la figure 4 est une vue agrandie de la pointe de la tige filetée du dispositif de vis de la figure 1,
- la figure 5 est une vue schématique d'un mode de réalisation simplifié du dispositif de vis à module d'insertion cassable selon l'invention,
- la figure 6 est une vue selon la flèche G de la figure 5,
- la figure 7 est une vue d'un exemple de vis autocassable selon l'art antérieur,
- la figure 8 est une vue d'ensemble d'un outil d'extraction de vis à module d'insertion cassable selon un aspect de l'invention,
- la figure 9 est une vue de dessus de l'outil d'extraction de la figure 8,
- la figure 10 est une vue de dessous de l'outil d'extraction de la figure 8, et
- la figure 11 est une vue agrandie de la partie tubulaire terminale de l'outil d'extraction de la figure 8.

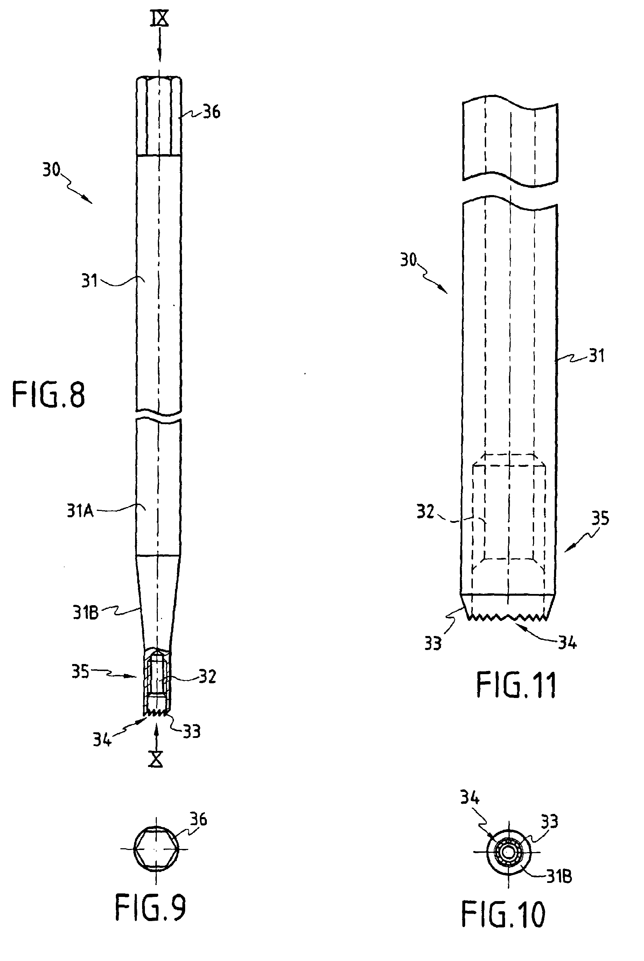

On décrira d'abord brièvement en référence à la figure 7 un exemple de cheville ou de vis autocassable selon l'art antérieur.

La vis autocassable 110 comprend une tige de préhension 101 constituée par un embout d'adaptation dans le mandrin d'un outil d'entraînement en rotation, qui peut être de type mécanique, électrique ou pneumatique.

La tige de préhension 101 est reliée par une portion tronconique 102 et un étranglement 103 à la tête 105 d'une vis 104. L'étranglement 103 constitue une zone de moindre résistance à un couple donné, de sorte que lorsque la tête de vis 105 vient en butée et que l'effort de torsion exercé sur l'embout 101 dépasse un seuil prédéterminé, il se produit une rupture au niveau de l'étranglement 103.

La vis 104 comprend sous la tête 105 une première partie cylindrique non filetée puis une portion filetée 106 prolongée par une partie munie d'une entaille 107 de taraudage, elle-même prolongée par une portion tronconique 183 à laquelle est raccordée une extrémité perforante comportant deux faces planes opposées 181 taillées pour former une pointe 108.

La tête de vis 105 est fraisée et munie d'entailles diamétralement opposées permettant d'y accoupler un outil pour terminer le vissage manuel ou procéder à un dévissage de la vis lorsque celle-ci doit être retirée.

La vis autocassable 110 est réalisée en un matériau métallique biocompatible tel que l'acier inoxydable ou du TA6V, ou le cas échéant en un matériau minéral biointégrable ou encore en un matériau biocompatible et biodégradable.

Comme on l'a indiqué plus haut, le fait que la séparation entre la vis proprement dite et la tige de préhension s'opère par rupture en cisaillement lorsque l'effort de torsion devient trop grand constitue un inconvénient dans la mesure où l'opérateur ne peut pas choisir lui-même le point de rupture et où cette technique n'est pas universelle et en particulier ne s'applique pas correctement dans le cas où l'on doit effectuer une exostosectomie ou lorsque l'os cortical est de mauvaise qualité.

Par ailleurs, la présence de la tête de vis 105, même fraisée, constitue une gêne dans la mesure où elle ne peut être complètement enfouie, et le retrait de la vis s'avère délicat même lorsque la tête de vis 105 est munie d'entailles ou empreintes.

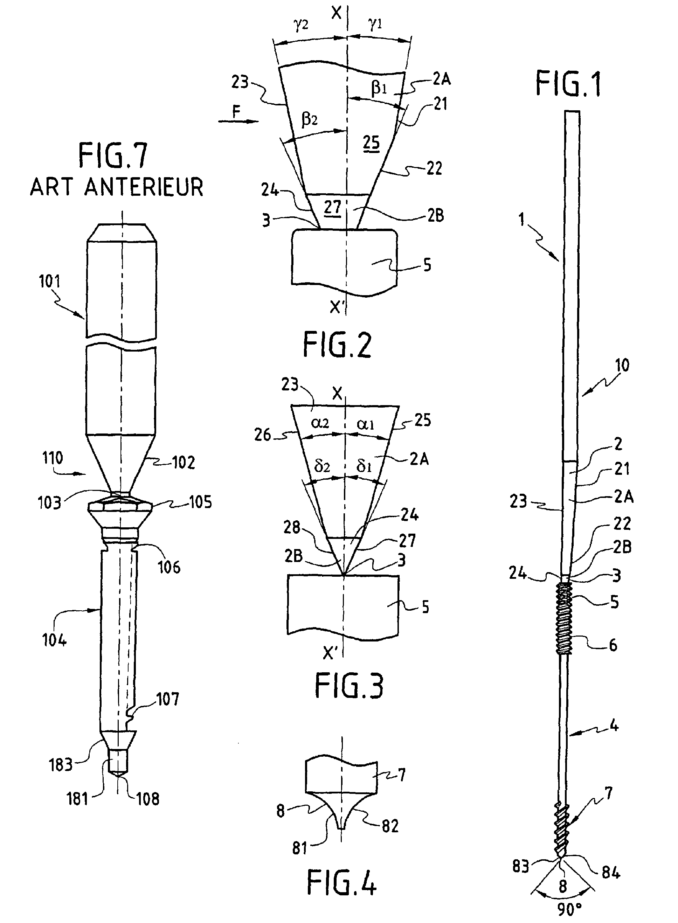

On décrira maintenant en référence aux figures 1 à 4 un premier exemple de dispositif de vis à module d'insertion cassable conforme à l'invention.

Un tel dispositif de vis 10 comprend essentiellement une tige de préhension 1 et une tige filetée implantable 4 dépourvue de tête de vis. La tige de préhension 1 comprend une portion 2 en forme de sifflet résistant à la torsion et raccordée à la tige filetée 4 au niveau d'un étranglement 3.

Le sifflet 2 et l'étranglement 3 qui définissent la jonction entre la tige de préhension 1 et la tige filetée 4 sont réalisés de telle manière que la tige de préhension 1 puisse être cassée et séparée de la tige filetée 4 au niveau de l'étranglement 3 par simple flexion volontaire de la tige de préhension 1 par rapport à la tige filetée 4.

La tige filetée 4, qui présente une extrémité distale 8 en forme de pointe, est d'abord solidaire de la tige de préhension 1 en étant raccordée à celle-ci par l'étranglement 3 et le sifflet 2. La tige de préhension 1 peut être introduite dans le mandrin d'un outil d'entraînement en rotation de type électrique, mécanique ou pneumatique. Lorsque la tige de préhension 1 est entraînée en rotation, la tige filetée 4 est elle-même introduite dans l'os et engagée en profondeur sans risque de rupture de la liaison entre la tige filetée 4 et la tige de préhension 1 dans la mesure où l'étranglement 3 est réalisé de manière à résister à un couple de torsion.

Lorsque la tige filetée 4 est introduite complètement de telle manière que l'étranglement 3 affleure, après un contrôle visuel du bon positionnement de la tige filetée 4 et de la qualité de l'ostéosynthèse, l'opérateur peut retirer le mandrin dans lequel est engagée la tige de positionnement 1 et par une simple flexion de la tige de positionnement 1 provoque la rupture de celle-ci au niveau de l'étranglement 3. La tige filetée 4 reste implantée dans la position qui a été précédemment contrôlée visuellement par l'opérateur. La rupture au niveau de l'étranglement 3 s'effectue ainsi de façon contrôlée par l'opérateur et non pas en fonction du dépassement d'un couple donné lors de l'opération de vissage.

Contrairement aux dispositifs de l'art antérieur, il est ainsi garanti que la tige filetée 4 pourra être engagée complètement dans l'os sans rupture prématurée et qu'à l'inverse, en cas de mauvaise qualité de l'os, la tige filetée 4 n'entraînera pas la destruction de l'os proximal par perforation, suite à une absence de rupture au niveau de l'étranglement 3.

La portion 2 en forme de sifflet comprend une partie principale 2A prolongée par une partie biseautée 2B du côté raccordé à la tige filetée 4 au niveau de l'étranglement 3.

Le sifflet 2 peut présenter différentes formes en fonction des applications.

Sur les figures 1 à 3, on a représenté un sifflet dissymétrique (fig. 2) par rapport à un plan axial de la tige de préhension 1.

Si l'on considère essentiellement les figures 2 et 3, on voit un sifflet 2 comprenant deux faces principales 25, 26 inclinées respectivement d'un angle α₁, α₂ par rapport à l'axe X X' de la tige de préhension, et deux faces latérales 21, 23 qui peuvent elles-mêmes être inclinées respectivement d'un angle γ₁, γ₂ par rapport à l'axe X X'.

Le biseau 2B présente deux faces principales 22, 24 inclinées respectivement d'un angle β₁, β₂ par rapport à l'axe X X' de la tige de préhension 1. Les deux faces latérales 27, 28 du biseau 2B peuvent présenter le même angle α₁, α₂ que les faces principales 25, 26 du corps du sifflet 2A ou un angle un peu plus grand δ₁, δ₂ (fig. 3). Les angles β₁, β₂ des faces principales 22, 24 du biseau 2B par rapport à l'axe X X' sont plus grands que les angles γ₁, γ₂ des faces latérales 21, 23 du sifflet 2. Toutefois, comme on peut le voir sur la figure 2, il peut y avoir dissymétrie et par exemple la face principale 22 du biseau 2B peut être plus rapprochée de l'axe X X' que la face principale 24 et peut également se prolonger plus haut dans la partie principale 2A.

Les angles α₁, α₂, β₁, β₂, γ₁, γ₂, δ₁, δ₂, peuvent être compris entre environ 15° et 30°.

Les angles β₁, β₂ peuvent par exemple être de l'ordre de 20° tandis que les angles α₁, α₂ peuvent être par exemple de l'ordre de 15°.

Selon un aspect important de l'invention, la tige filetée 4 comprend un filetage proximal croisé avec un premier filetage 6 à droite et un deuxième filetage 5 à gauche formant contre-filet. Le deuxième filetage 5 est superposé au premier filetage 6 au moins dans la partie voisine de l'étranglement 3. Le deuxième filetage 5 peut ainsi s'étendre par exemple sur la moitié de la hauteur du premier filetage 6 ou sur toute la hauteur du premier filetage 6.

Un filetage distal 7 peut être différent du filetage proximal croisé 5, 6.

Ainsi, les filetages proximal 5, 6 et distal 7 peuvent présenter des pas différents de manière à être compressifs.

Le filetage proximal croisé 5, 6 peut être de type à profil cortical, tandis que le filetage distal 7 est de type à profil spongieux.

La partie terminale pointue 8 de la tige filetée 4 peut elle-même comprendre deux surfaces concaves 81, 82 délimitées par deux parois latérales 83, 84 définissant entre elles un angle de 90°. Toutefois, d'autres formes d'extrémité pointue sont également envisageables.

La présence d'un filetage proximal croisé, avec un premier filetage droit 6 et un deuxième filetage gauche 5 permet une bonne repousse osseuse sur le métal de la tige filetée. Avantageusement, le deuxième filetage gauche 5 n'est formé que sur la partie haute du premier filetage droit 6.

Grâce à la présence du deuxième filetage gauche 5, il est possible d'extraire facilement la vis sans tête 4 grâce à un outil d'extraction qui sera décrit plus loin, l'outil d'extraction étant simplement introduit sur le deuxième filetage gauche 5 dans le sens du dévissage de façon à se visser d'abord sur ce deuxième filetage 5 puis à entraîner l'ensemble de la tige filetée 4 dans le sens du dévissage par rapport à l'os.

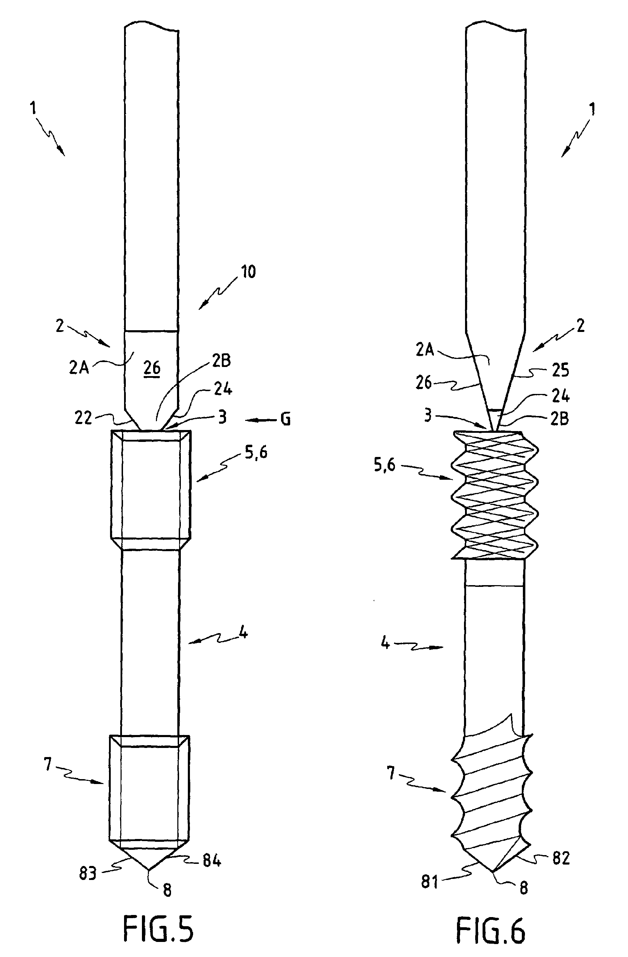

Les figures 5 et 6 montrent une réalisation simplifiée d'un dispositif de vis 10 à module d'insertion cassable selon l'invention, dans lequel le sifflet 2 comprend une partie principale 2A avec des faces latérales qui sont essentiellement parallèles à l'axe de la tige de positionnement 1, seules les faces principales 25, 26 du sifflet 2 étant inclinées par rapport à l'axe de la tige de positionnement 1. Dans le cas du mode de réalisation des figures 5 et 6, le sifflet 2 et son biseau 2B sont également réalisés de façon symétrique par rapport à un plan passant par l'axe de la tige de positionnement 1. Les autres caractéristiques du dispositif de vis des figures 5 et 6 sont identiques à celles décrites en référence aux figures 1 à 4 et les éléments correspondants portent les mêmes références.

On décrira maintenant en référence aux figures 8 à 11 un outil 30 d'extraction d'un dispositif de vis 10 selon l'invention.

L'outil d'extraction 30 comprend une tige d'extraction 31 dont la portion distale constitue une partie tubulaire 35 comprenant une partie lisse d'extrémité prolongée par un trou taraudé 32 muni d'un filetage à gauche correspondant au deuxième filetage à gauche 5 de la vis à extraire 10.

La partie tubulaire 35 est avantageusement terminée par une extrémité dentelée 34 munie d'un biseau 33. Le corps 31A de la tige d'extraction 31 peut présenter une section supérieure à la section de la partie tubulaire 35 et être relié à cette dernière par une partie tronconique 31B.

A l'extrémité opposée à la partie tubulaire 35, l'outil d'extraction comprend une partie 36 d'emmanchement dans un mandrin d'entraînement en rotation.

Pour l'extraction d'une vis ou tige filetée 4 implantée dans un os, on procède au repérage du filetage proximal 5, 6 de cette vis sans tête et on introduit la partie tubulaire 35 de l'outil d'extraction 30 autour de ce filetage proximal 5, 6. L'extrémité biseautée et dentelée de la partie tubulaire 35 facilite de dégagement du filetage proximal 5, 6 et la partie lisse d'extrémité de la partie tubulaire 35 facilité le positionnement et l'alignement de l'outil d'extraction 30 par rapport à la tige filetée 4. Le trou taraudé 32 de la partie tubulaire 35 est alors vissé, dans le sens du dévissage, sur le filetage 5 à pas à gauche de la tige filetée 4, jusqu'en butée.

L'extrémité 36 de l'outil d'extraction 30 est alors emmanchée dans le mandrin d'un outil d'entraînement en rotation. Par action de dévissage, la partie tubulaire 35 ou nez de l'outil d'extraction en prise avec le filetage 5 de la tige filetée entraîne en rotation dans le sens du dévissage par rapport à l'os l'ensemble de la tige filetée 4 jusqu'à extraction complète. La tige filetée peut alors être retirée de l'outil d'extraction 30 et l'outil d'extraction 30 est lui-même dissocié du mandrin engagé sur l'extrémité 36.

L'opération d'extraction d'une vis sans tête implantée peut ainsi s'opérer de façon simple, rapide et sûre à l'aide d'un outillage de conception simple.

Par ailleurs, on notera que lors de l'opération d'insertion, les caractéristiques du dispositif de vis à module d'insertion cassable selon l'invention garantissent que la cassure réalisée au moment précis choisi par l'opérateur est nette et sans bavure, et de ce fait totalement atraumatique.

## Revendications

1. Dispositif de vis à module d'insertion cassable pour des techniques d'ostéosynthèse, comprenant une tige filetée implantable (4) raccordée par un étranglement (3) à une tige de préhension (1), **caractérisé en ce que** la tige de préhension (1) comprend une portion (2) en forme de sifflet résistant à la torsion et raccordée, au niveau de l'étranglement (3), à la tige filetée (4) dépourvue de tête de vis, de telle manière que la tige de préhension (1) puisse être cassée et séparée de la tige filetée (4) au niveau de l'étranglement (3) par simple flexion volontaire de la tige de préhension (1) par rapport à la tige filetée (4).

2. Dispositif de vis selon la revendication 1, **caractérisé en ce que** la portion (2) en forme de sifflet est munie d'un biseau (2B) du côté raccordé à la tige filetée (4) au niveau de l'étranglement (3).

3. Dispositif de vis selon la revendication 1 ou la revendication 2, **caractérisé en ce que** le sifflet (2) présente une forme symétrique par rapport à un plan axial de la vis (10).

4. Dispositif de vis selon la revendication 1 ou la revendication 2, **caractérisé en ce que en ce que** le sifflet (2) présente une forme dissymétrique par rapport à un plan axial de la vis (10).

5. Dispositif de vis selon l'une quelconque des revendications 1 à 4, **caractérisé en ce que** le sifflet (2) présente des parois (25, 26 ; 21, 23) inclinées d'un angle (α₁, α₂, γ₁, γ₂) compris entre 15° et 30° par rapport à l'axe de la vis (10).

6. Dispositif de vis selon l'une quelconque des revendications 1 à 5, **caractérisé en ce que** la tige filetée (4) comprend au moins un filetage proximal croisé (5, 6) avec un premier filetage (6) à droite, et un deuxième filetage (5) à gauche formant contre-filet superposé au premier filetage (6) au moins dans la partie voisine de l'étranglement (3), et un filetage distal (7) au voisinage de la partie terminale pointue (8) de la tige filetée (4).

7. Dispositif de vis selon la revendication 6, **caractérisé en ce que** le filetage proximal croisé (5, 6) et le filetage distal (7) sont différents.

8. Dispositif de vis selon la revendication 7, **caractérisé en ce que** les filetages proximal (5, 6) et distal (7) présentent des pas différents de manière à être compressifs.

9. Dispositif selon la revendication 7, **caractérisé en ce que** le filetage proximal croisé (5, 6) est de type à profil cortical tandis que le filetage distal (7) est de type à profil spongieux.

10. Dispositif de vis selon l'une quelconque des revendications 6 à 9, **caractérisé en ce que** le deuxième filetage (5) à gauche formant contre-filet s'étend sur une partie seulement de la hauteur du premier filetage (6) à droite.

11. Dispositif de vis selon l'une quelconque des revendications 6 à 9, **caractérisé en ce que** le deuxième filetage (5) à gauche formant contre-filet s'étend sensiblement sur toute la hauteur du premier filetage (6) à droite.

12. Dispositif de vis selon l'une quelconque des revendications 1 à 11, **caractérisé en ce que** la partie terminale pointue (8) de la tige filetée (4) comprend deux surfaces concaves (81, 82) délimitées par deux parois latérales (83, 84) définissant entre elles un angle de 90°.

13. Outil d'extraction d'un dispositif de vis selon l'une quelconque des revendications 6 à 11, **caractérisé en ce qu'**il comprend une tige d'extraction (31) dont la portion distale constitue une partie tubulaire (35) comprenant une partie lisse d'extrémité prolongée par un trou taraudé (32) muni d'un filetage à gauche correspondant au deuxième filetage (5) à gauche formant contre-filet de la vis (10) à extraire, la partie tubulaire (35) étant terminée par une extrémité dentelée et biseautée.

14. Outil d'extraction selon la revendication 13, **caractérisé en ce que** le corps (31A) de la tige d'extraction (31) présente une section supérieure à la section de la partie tubulaire (35), et est relié à cette dernière par une partie tronconique (31B).

15. Outil d'extraction selon la revendication 13 ou la revendication 14, **caractérisé en ce qu'**il comprend à son extrémité opposée à la partie tubulaire (35) une partie (36) d'emmanchement dans un mandrin d'entraînement en rotation.
